Europäisches Patentamt

European Patent Office

Office européen des brevets

(19)

(11)  EP 1 215 501 A1

(12)  EUROPEAN PATENT APPLICATION

(43) Date of publication:
19.06.2002  Bulletin 2002/25

(51) Int Cl.⁷: $G01N\ 33/58$

(21) Application number: 01310076.3

(22) Date of filing: 30.11.2001

(84) Designated Contracting States:
AT BE CH CY DE DK ES FI FR GB GR IE IT LI LU
MC NL PT SE TR
Designated Extension States:
AL LT LV MK RO SI

(30) Priority: 30.11.2000  US 250681

(71) Applicant: MOLECULAR DEVICES
CORPORATION
Sunnyvale, California 94089 (US)

(72) Inventor: Twu, Jesse J.
Cupertino, California 95014 (US)

(74) Representative: Harrison Goddard Foote
Belgrave Hall
Belgrave Street
Leeds LS2 8DD (GB)

(54)  **Use of a poly(amino-acid)-metal ion complex to link a label to a species of interest**

(57)  Systems, including compositions and methods, for purifying and/or labeling proteins or other molecules of interest and/or for assaying the conformational and/or binding states of such molecules. The compositions may include products having the formula

T-P-M-L

where T is a species, M is a metal ion, P is a peptide or protein that binds the metal ion, and L is a luminescent label. The methods may include purifying and/or labeling a molecule of interest, detecting luminescence energy transfer, detecting dissociation and/or association of a molecule or molecules of interest, detecting a conformational change in a molecule of interest, and detecting an analyte, among others.

EP 1 215 501 A1

**Description**

<u>Cross-Reference to Related Applications</u>

This application is based upon and claims the benefit under 35 U.S.C. § 119 of the following U.S. provisional patent application, which is incorporated herein by reference in its entirety for all purposes: Serial No. 60/250,681, filed November 30, 2000.

This application incorporates by reference in their entirety for all purposes the following U.S. Patents: No. 5,355,215, issued October 11, 1994; and No. 6,097,025, issued August 1, 2000.

This application incorporates by reference in their entirety for all purposes the following published European patent applications: Publication No. EU/1101113, published May 23, 2001; and Publication No. EU/1156329, published November 21, 2001.

This application incorporates by reference in their entirety for all purposes the following PCT patent applications: Serial No. PCT/IB00/00095, filed January 26, 2001; and Serial No. PCT/IB00/00097, filed January 26, 2001.

This application incorporates by reference in their entirety for all purposes the following published PCT patent applications: Publication No. WO99/23466, published May 14, 1999; Publication No. WO99/31503, published June 24, 1999; Publication No. WO99/37203, published July 29, 1999; Publication No. WO99/42817, published August 26, 1999; Publication No. WO99/54711, published October 28, 1999; Publication No. WO00/04364, published January 27, 2000; Publication No. WO00/05336, published February 3, 2000; Publication No. WO00/06989, published February 10, 2000; Publication No. WO00/06990, published February 10, 2000; Publication No. WO00/06991, published February 10, 2000; Publication No. WO00/23785, published April 27, 2000; Publication No. WO01/34764, published May 17, 2001; Publication No. WO00/42209, published July 20, 2000; Publication No. WO00/47693, published August 17, 2000; Publication No. WO00/50877, published August 31, 2000; Publication No. WO00/55372, published September 21, 2000; Publication No. WO00/66269, published November 9, 2000; Publication No. WO00/75664, published December 14, 2000; and Publication No. WO01/04608, published January 18, 2001.

This application incorporates by reference in their entirety for all purposes the following U.S. Patent Applications: Serial No. 09/337,623, filed June 21, 1999; Serial No. 09/596,444, filed June 19, 2000; Serial No. 09/710,061, filed November 10, 2000; Serial No. 09/722,247, filed November 24, 2000; Serial No. 09/836,575, filed April 16, 2001; Serial No. 09/934,348, filed August 20, 2001; Serial No. 09/952,461, filed September 14, 2001; Serial No. 09/957,116, filed September 19, 2001; Serial No. _____, filed October 29, 2001, titled LIGHT DETECTION DEVICE, and naming Joseph H. Jackson III, Dean G. Hafeman, and Todd French as inventors; and Serial No. _____, filed November 12, 2001, titled APPARATUS AND METHODS FOR TIME-RESOLVED OPTICAL SPECTROSCOPY, naming Todd E. French, John C. Owicki, and Douglas N. Modlin as inventors.

This application incorporates by reference in their entirety for all purposes the following U.S. Provisional Patent Applications: Serial No. 60/223,642, filed August 8, 2000; Serial No. 60/232,365, filed September 14, 2000; Serial No. 60/233,800, filed September 19, 2000; Serial No. 60/244,012, filed October 27, 2000; Serial No. 60/250,683, filed November 30, 2000; Serial No. 60/267,639, filed February 10, 2001; Serial No. 60/287,697, filed April 30, 2001; Serial No. 60/309,800, filed August 2, 2001; Serial No. 60/316,704, filed August 31, 2001; Serial No. 60/318,038, filed September 7, 2001; Serial No. 60/318,149, filed September 7, 2001; and Serial No. 60/322,178, filed September 13, 2001.

This application incorporates by reference in its entirety for all purposes the following publications: Richard P. Haugland, *Handbook of Fluorescent Probes and Research Chemicals* (6th ed. 1996); and Joseph R. Lakowicz, PRINCIPLES OF FLUORESCENCE SPECTROSCOPY (2nd Ed. 1999).

<u>Field of the Invention</u>

The invention relates to systems for purifying and/or labeling species of interest and/or for assaying the conformational and/or binding states of such species.

<u>Background of the Invention</u>

Medical, biological, and pharmaceutical researchers face the recurring problem of purifying molecules of interest and labeling them for use in various assays, including assays to determine whether two molecules interact with each other.

Radioactivity has been used extensively to label molecules, but it has serious drawbacks. First, researchers are exposed to the harmful effects of radiation, and disposal of radioactive waste is problematic. Second, radioactive isotopes have limited half-lives, and, therefore, radioactively labeled molecules may need to be relabeled frequently. Finally, radioactive labeling by itself yields no information regarding the physical proximity of the labeled molecule to other molecules.

**EP 1 215 501 A1**

Luminescence labeling remedies these drawbacks. First, luminescent labels present no significant health risk to researchers and pose no significant disposal problem. Second, luminescent labels do not have limited half-lives, and, therefore, frequent relabeling is unnecessary. Finally, luminescent labeling can yield information regarding the physical proximity of the labeled molecules to other molecules by the use of luminescence resonance energy transfer or luminescence polarization, among others.

Luminescence resonance energy transfer can be used to detect physical proximity of two molecules. This method utilizes a luminescent donor and a matched acceptor. The luminescent donor preferably is capable of excitation by a wavelength of light that does not significantly excite the acceptor, and the emission spectrum of the donor should overlap with the excitation spectrum of the acceptor. To measure the physical proximity of two molecules, a donor is attached to one molecule, and an acceptor is attached to the other. The labeled molecules are combined. The donor is excited by light of an appropriate wavelength. If the acceptor is sufficiently close to the donor, energy will be transferred, and the acceptor (if luminescent) will emit light. A decrease in donor luminescence and lifetime and an increase in acceptor luminescence are all quantifiable indices of physical proximity between the donor and acceptor.

Luminescence polarization also can be used to detect physical proximity. When a luminophore is excited by irradiation with light of a wavelength that it can absorb, there is a finite time between the absorption of the light and the emission of light of a different wavelength. This time period, called a luminescence lifetime, varies among different luminophores. If the light used to excite the luminophore is polarized, it preferentially excites only luminophores having absorption dipoles aligned parallel to the polarization of the excitation light. These molecules will emit light polarized parallel to their emission dipoles. This means that the emitted light will be polarized as long as the luminophores have not physically rotated during the luminescence lifetime. To the extent that some or all of these molecules have rotated during the luminescence lifetime, the emitted light will be less polarized. The speed with which a molecule rotates is inversely correlated with its molecular weight: that is, the larger the molecule, the more slowly it rotates. Thus, when a small molecule conjugated to a luminophore is bound by a large molecule, such as a protein, the extent of luminescence polarization can increase upon binding. This increase in luminescence polarization can be used to detect binding of small molecules by large ones.

Luminescence resonance energy transfer and luminescence polarization are described in further detail in the patents, patent applications, and nonpatent references cited under Cross-References and incorporated herein by reference.

Past methods for incorporating luminescent labels into molecules of interest have clear disadvantages. They generally rely on antigen/antibody interactions. Such interactions may not always be of high affinity. Moreover, like other proteins, antibodies may lose activity, in this case, the ability to bind antigen, during storage. Finally, creating luminescently labeled antibodies via a chemical reaction to attach a luminophore may not be straightforward.

In one method, a protein tag that can be recognized by an available, luminescently labeled antibody is incorporated into a protein of interest, and a luminescently labeled antibody serves as a label for the protein of interest. This necessitates inserting nucleic acid sequences encoding the protein to be labeled into a vector that contains nucleic acid sequences encoding the protein tag. The sequences must be positioned in such a way that, upon translation, the protein tag will be appended to either end of the protein of interest. The luminescently labeled antibody used to detect the presence of the protein tag must be purchased or manufactured.

Alternatively, in another method, an antibody is raised against a molecule of interest and luminescently labeled. Raising an antibody is a time-consuming process that involves, at a minimum, injecting an animal, such as a mouse or a rabbit, with an antigen and later collecting antibody-rich serum. This serum contains a mixture of (polyclonal) antibody molecules and may be purified further if desired. To obtain a source of pure (monoclonal) antibody, single antibody-producing cells with a limited life span are fused with single cells from an immortal cell line. The resulting immortal cell hybrids are screened to determine whether they produce an antibody that reacts with the antigen of interest. A luminescent label is chemically attached to this antibody, and the labeled antibody is then used to detect the protein. As an alternative to chemically conjugating a luminescent label to this antibody, an available, luminescently labeled antibody that recognizes a conserved portion of the antibody is used to detect the antibody and thus the molecule of interest.

Summary of the Invention

The invention provides systems, including methods and compositions, for purifying and/or labeling molecules or species of interest and/or for assaying the conformational and/or binding states of such molecules. In one aspect, the invention provides products which use a poly(amino acid)-metal ion complex to link a label to a species of interest. (The term "complex" is not used here to indicate a particular type of linkage between the ion and the poly(amino acid) but to refer to a species having a metal ion and a poly(amino acid) in association.)

3

Brief Description of the Drawings

Figure 1 is a schematic representation of the process of purifying and labeling a protein.

Figure 2 is a schematic representation of the process of labeling any kind of molecule.

Figure 3 is a schematic representation of the process of detecting physical proximity of two labeled molecules using luminescence resonance energy transfer.

Figure 4 is a schematic representation of the process of detecting binding of a labeled small molecule by a macromolecule using luminescence polarization.

Detailed Description of the Invention

The invention provides systems, including compositions and methods, for purifying and/or labeling species (typically molecules) of interest and/or for assaying the conformational and/or binding states of such species. The compositions include labeled species of interest, such as labeled members of a luminescence energy transfer donor-acceptor pair. The methods may include purifying and/or labeling a species of interest, detecting luminescence energy transfer, detecting dissociation and/or association of a molecule or molecules of interest, detecting a conformational change in a molecule of interest, and detecting an analyte, among others.

These and other aspects of the invention are described in the following sections: (I) compositions, (II) methods for purifying and labeling proteins, (III) methods for purifying and labeling any kind of molecule, (IV) overview of selected methods for detecting conformational state and/or proximity of molecules, (V) energy transfer methods for detecting conformational state and/or proximity of molecules, and (VI) polarization methods for detecting conformational state and/or proximity of molecules.

*I. Compositions*

The labeled products of the invention have the formula

$$T\text{-}P\text{-}M\text{-}L$$

where T is a labeled species or molecule of interest, M is a metal ion, P is a peptide or protein that binds the metal ion, and L is a luminescent label. The peptide or protein P associates with the metal ion M by forming a covalent or noncovalent complex with the metal ion. Similarly, luminescent label L is associated with metal ion M via covalent or noncovalent interactions that optionally include any of a variety of chelating groups or intervening linkages.

The labeled species may be provided and/or used alone or in combination with other materials in the form of kits. The kits may include any additional reagents useful or required for a particular application. These additional reagents may include a buffering agent, a luminescence calibration standard, an enzyme, an enzyme substrate, a nucleic acid stain, or a labeled antibody. The kits may be configured for use in conjunction with a sample in a multiwell microplate or a microfluidic chip, for example, by being divided or divisible into aliquots corresponding to the number of wells in the microplate (e.g., 96, 384, 1536, etc.).

These and other aspects of the compositions of the invention are described in the following sections: (A) labeled species T, (B) peptide or protein P, and (C) luminescent label L.

*I.A Labeled Species T*

The species T which is labeled may be any product of interest that is substantially stable and synthetically accessible. Here, and throughout this application, species is used generally to denote individual atoms or sets of like or unlike atoms, such as molecules, held together by chemical forces, including covalent bonds, noncovalent bonds, hydrogen bonds, ionic bonds, hydrophobic interactions, hydrophilic interactions, van der Waals interactions, and the like. Thus, species might refer to a nucleotide, such as ATP, to a nucleic acid polymer, such as DNA, or to a nucleic acid polymer / protein complex, such as chromatin or a ribosome. Examples of particularly useful labeled molecules include amino acids, peptides, proteins, nucleotides, nucleosides, oligonucleotides, nucleic acid polymers, carbohydrates, lipids, and non-biological polymers. Alternatively, the species of interest is a component of a cell, a cellular system, a cellular fragment, or a subcellular particle. Examples include, among others, virus particles, bacterial particles, virus components, biological cells (such as animal cells, plant cells, bacteria, yeast, or protists), or cellular components.

In one aspect of the invention, the species is an amino acid, peptide, protein, polysaccharide, nucleotide, oligonucleotide, or nucleic acid polymer. In another aspect of the invention, the species is a drug, a lipid, a lipid assembly, a non-biological organic polymer, or a polymeric microparticle. In one embodiment, conjugates of biological polymers such as peptides, proteins, oligonucleotides, nucleic acid polymers are also labeled with an additional luminescent of non-luminescent dye, to form a luminescence energy-transfer pair.

In particular, the species is optionally an amino acid (including those that are protected or are substituted by phosphates, carbohydrates, or carboxylic acids), or is a polymer of amino acids such as a peptide or protein. Preferred conjugates of peptides contain at least 5 amino acids, more typically 5 to 36 amino acids. Suitable peptides include, but are not limited to, neuropeptides, cytokines, toxins, protease substrates, and protein kinase substrates. Suitable protein conjugates include enzymes, antibodies, lectins, glycoproteins, histones, albumins, lipoproteins, avidin, streptavidin, protein A, protein G, phycobiliproteins and other fluorescent proteins, hormones, toxins, and growth factors. Alternatively, the conjugated protein is an antibody, an antibody fragment, avidin, streptavidin, a toxin, a lectin, a hormone, or a growth factor. Where the species is a toxin, it may be a neuropeptide or a phallotoxin, such as phalloidin.

Alternatively, the species is a nucleic acid base, nucleoside, nucleotide or a nucleic acid polymer, including those that are modified to possess an additional linker or spacer for attachment of peptide or protein P.

Suitable oligonucleotide or nucleic acid polymer conjugates include single- or multi-stranded, natural or synthetic DNA or RNA, or DNA/RNA hybrids, or incorporate an unusual linker such as morpholine derivatized phosphates, or peptide nucleic acids such as N-(2-aminoethyl)glycine units. When the nucleic acid is an oligonucleotide, it typically contains fewer than 50 nucleotides, more typically fewer than 25 nucleotides.

In another aspect of the invention, the species is a carbohydrate that is typically a polysaccharide, such as a cextran, FICOLL, heparin, glycogen, amylopectin, mannan, inulin, starch, agarose, and cellulose, but that also may be a lipopolysaccharide, among others. Preferred polysaccharide conjugates are dextran or FICOLL conjugates.

In yet another aspect of the invention, the species is a lipid, such as a glycolipid, phospholipid, sphingolipid, or steroid. Alternatively, the species may be incorporated into a lipid assembly, such as a liposome.

The species is optionally an organic or inorganic polymer, such as a polymeric film, polymeric wafer, polymeric membrane, polymeric particle, polymeric microparticle including magnetic and non-magnetic microspheres, conducting and non-conducting metals and non-metals, and glass and plastic surfaces and particles. In another aspect, the species is a glass or silica, which may be incorporated into an optical fiber or other structure.

In one embodiment, the species is a member of a specific binding pair, and is useful as a probe for the complementary member of that specific binding pair. Members of a specific binding pair typically incorporate an area on their surface or in a cavity that specifically binds to and is complementary with a particular spatial and polar organization of the other. Preferred specific binding pair members are proteins that bind noncovalently to low molecular weight ligands, such as biotin or drug-haptens. Representative specific binding pairs are shown in Table 1.

Table 1

| Representative Specific Binding Pairs | |
|---|---|
| antigen | antibody |
| biotin | avidin (or streptavidin or anti-biotin) |
| IgG* | protein A or protein G |
| drug | drug receptor |
| toxin | toxin receptor |
| carbohydrate | lectin or carbohydrate receptor |
| peptide | peptide receptor |
| protein | protein receptor |
| enzyme substrate | enzyme |
| DNA (RNA) | aDNA (aRNA)** |
| hormone | hormone receptor |

\* IgG is an immunoglobulin

\*\* aDNA and aRNA are the antisense (complementary) strands used for hybridization

I.B. <u>Peptide or Protein P</u>

The invention utilizes the binding interaction between some sequences of amino acids and selected metal ions. For example, polyhistidine binds to $Ni^{2+}$ or $Zn^{2+}$ ions. Binding of polyhistidine with $Ni^{2+}$ has been used to purify proteins having six consecutive histidines from complex mixtures of proteins. To do this, $Ni^{2+}$ is typically immobilized on a resin used to form a column, which is then used to purify proteins containing polyhistidine.

Phosphorylated phosphoproteins bind to some trivalent metal ions such as $Ga^{3+}$ or $Fe^{3+}$. This binding is not dependent on the amino acid sequence of the protein, only on phosphorylation of the protein. Because there are many phosphorylated proteins in a cell, phosphoprotein binding by metal ions is not especially useful for purifying a protein from a complex mixture of cellular proteins. However, phosphoprotein binding of metal ions could be used, for example, to detect protein phosphorylation or to purify a phosphoprotein from an in vitro translation mixture.

Peptide or protein P generally comprises any peptide or protein that, in a modified or unmodified state, can bind to the desired metal ion, M. Examples of protein tags include polyhistidine, peptides in which every other residue is a histidine, or peptides that can be phosphorylated. An example of a modification that may allow binding of a metal ion is phosphorylation of the peptide. Where P is a polyhistidine, M is preferably $Ni^{2+}$ or $Zn^{2+}$. Where P is a phosphoprotein, M is preferably $Ga^{3+}$ or $Fe^{3+}$.

Peptide or protein P is typically covalently bound to the molecule of interest T, although noncovalent association is an acceptable means of coupling P to molecule T, provided that the association is substantially stable. Conventional conjugation methods may be used to join peptide or protein P to molecule T; alternatively, where T is a peptide or protein, the vector used to express T may be modified to include the sequence coding for the desired peptide or protein P, so that expression of the vector results in a protein sequence that incorporates molecule T and peptide or protein P.

*I.C. Luminescent Label L*

The luminophore L is any luminescent moiety that is able to produce a luminescence response upon illumination with light of an appropriate wavelength, and is readily associated with the metal ion M. In one aspect of the invention, L incorporates one or more functional groups that associate with metal ion M, for example, by forming a coordination complex. Typical functional groups include carboxylic acids, hydroxyls, amines, thiols, and heterocyclic ring systems. L optionally incorporates one or more well-known metal-chelating groups, such as derivatives of ethylene diamine, ethylene diamine tetraacetic acid (EDTA), crown ethers, cryptands, phenanthrolines, pyridines, anilines, imidazoles, and/or benzimidazoles, among others. In one aspect of the invention, L incorporates a nitrilotriacetic acid derivative (NTA) that is capable of associating with an M that is a $Ni^{2+}$ ion.

In one aspect of the invention, the luminophore L incorporates a metal-ligand complex, such as those including ruthenium, osmium, or rhenium, or L incorporates a lanthanide ion, such as $Eu^{3+}$ or $Tb^{3+}$, that serves as a source of long-lifetime luminescence. Typically, where L incorporates an additional metal ion, L also includes a chelating group, distinct from any metal-chelating group selected to associate with metal ion M, suitable for complexing the desired metal ion.

The luminophore L may incorporate a synthetic dye that exhibits a fluorescence response upon illumination with light of an appropriate wavelength, such as, among others, a pyrene, an anthracene, a naphthalene, an acridine, a stilbene, an indole or benzindole, an oxazole or benzoxazole, a thiazole or benzothiazole, a 4-amino-7-nitrobenz-2-oxa-1,3-diazole (NBD), a cyanine, a carbocyanine, a merocyanine, a carbostyryl, a porphyrin, a salicylate, an ethidium, a propidium, an anthranilate, an azulene, a perylene, a pyridine, a quinoline, a coumarin, (including hydroxycoumarins and aminocoumarins as well as sulfonated and fluorinated derivatives thereof), a dipyrrometheneboron difluoride, a dibenzodipyrrometheneboron difluoride, a xanthene, an oxazine, a benzoxazine, a carbazine, and/or a phenalenone or benzphenalenone.

In a preferred aspect of the invention, the fluorophore is a benzimidazole, a phenoxazine, an ethidium, a propidium, an acridine, a styryl dye, a carbocyanine, a merocyanines, a coumarin, a pyrene (such as CASCADE BLUE dye), a chrysene, a stilbene, a carbazine, an anthracene, a naphthalene, a dipyrrometheneboron difluoride (such as a BODIPY dye), or a dibenzodipyrrometheneboron difluoride (such as a BENZOBODIPY dye).

More typically, the fluorophore is a xanthene dye, such as a fluorescein, a rhodol, or a rhodamine. Fluorescein, as used here, includes derivatives of fluorescein such as fluorinated fluorescein, benzo-substituted fluorescein, seminaphthofluorescein, or naphthofluorescein. Rhodol, as used here, includes seminaphthorhodafluors. Rhodamine, as used here, includes sulfonated rhodamines (such as TEXAS RED dye).

Additional examples of suitable luminophores are disclosed in the patents, patent applications, and nonpatent references listed above under Cross-References and incorporated herein by reference.

*II. Methods for Purifying and Labeling Proteins*

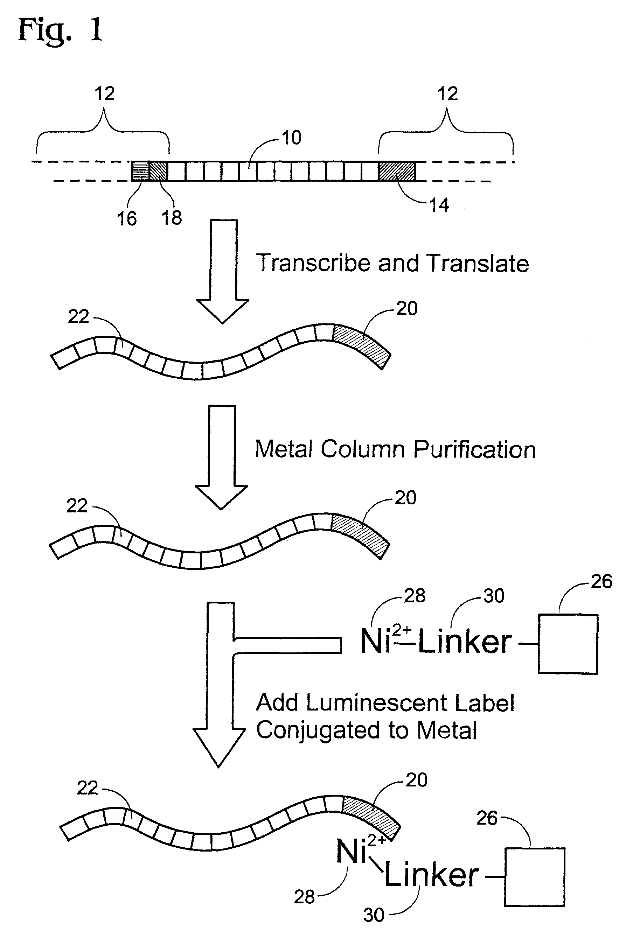

Figure 1 shows how a molecule can be purified and labeled using the affinity of a protein tag for a metal ion. A protein-encoding sequence 10 may be inserted in a nucleic acid vector 12 such that a nucleic acid sequence encoding a protein tag 14 is adjacent or within protein-encoding sequence 10. Nucleic acid sequence encoding a protein tag 14 is positioned such that it can be translated as part of the protein encoded by the protein-encoding sequence 10. Vector 12 includes sequences enabling transcription 16 and translation 18 of protein-encoding sequence 10 and sequence encoding the protein tag 14. The resulting hybrid protein 20, 22 includes protein sequences 22 encoded by protein-encoding sequence 10 and a protein tag 20 encoded by sequence encoding a protein tag 14. Protein tag 20, with or without modification, may bind to a metal ion 28. The hybrid protein 20, 22 may be purified using metal ions 28 that are immobilized on at least one solid support. To label hybrid protein 20, 22, a luminescent or enzymatically functional molecule 26 conjugated to metal ion 28 by means of a chemical linker 30 may be added to hybrid protein 20, 22, and the mixture incubated to allow binding. The binding of metal ion 28 to protein tag 20 may be tight enough to provide a label that will remain bound in a wide variety of conditions.

Protein-encoding sequence 10 generally comprises any nucleic acid sequence that encodes a protein. For example, it may include nucleic acid sequences encoding all or part of an enzyme, an antibody, a structural protein, or an intercellular signaling peptide such as a hormone.

Vector 12 generally comprises any agent for transferring genetic material from one location to another and preferably includes nucleic acid molecules that can be replicated in host cells. Suitable vectors include plasmids, cosmids, bacteriophage, viruses, and artificial chromosomes, among others. Host cells generally comprise cells that can be transformed with the vector and in which the vector can be propagated. These may include the following kinds of cells: bacteria, including *Escherichia coli;* yeast, including *Saccharomyces cerevisiae*; cultured cells originating from multicellular organisms; or unicellular eukaryotes. Transformation generally comprises the introduction of nucleic acids into host cells by any appropriate method. An example of transformation may include the introduction of any kind of vector nucleic acid into any host cells.

Nucleic acid sequence encoding a protein tag 14 generally comprises any nucleic acid sequence that encodes a peptide or protein P that binds to a metal ion in the presence or absence of further covalent modification to the peptide or protein. Examples of such sequences may include sequences encoding polyhistidine, sequences encoding a peptide in which every other residue is a histidine, or sequences encoding a protein that can be phosphorylated.

Sequences enabling transcription 16 generally comprise any sequence that promotes the initiation of transcription either in vivo or in vitro. Suitable sequences may include commonly available promoter sequences, such as the T7 or SP6 promoters, that function in vitro and also may include sequences that function to promote transcription in vivo in host cells in which vector 12 is propagated.

Sequences enabling translation 18 generally comprise any sequence that allows the initiation of translation either in vivo or in vitro. Sequences enabling translation 18 may include translational initiation sites that function in any in vitro translation system, such as *Escherichia coli* or rabbit reticulocyte in vitro translation systems. Sequences enabling translation 18 also may include translational initiation sites that function in vivo in any host cell in which vector 12 is propagated.

Hybrid protein 20, 22 generally comprises any protein 22 covalently connected to any protein tag 20. Examples of protein 22 may include, among others, all or part of an enzyme, an antibody, a structural protein, or a signaling peptide such as a hormone. The protein tag generally comprises any peptide that, in a modified or unmodified state, can bind to a metal ion.

Purification of hybrid protein 20, 22 using metal ions 28 attached to at least one solid support generally comprises any kind of solid support and any combination of steps that includes (1) incubating the hybrid protein 20, 22 with metal ions 28 attached to at least one solid support to allow binding of hybrid protein 20, 22 to metal ions 28, (2) washing metal ions 28 attached to at least one solid support to remove any unbound molecules, and (3) treating bound hybrid protein 20, 22 so that it is released from metal ions 28 attached to at least one solid support

Luminescent or enzymatically functional molecule 26 generally comprises any molecule that emits light in response to excitation by light of an appropriate wavelength or any molecule that catalyzes a reaction that produces a detectable change, such as a reaction with a chromogen that causes a color change.

Metal ion 28 generally comprises any metal ion in any state of oxidation, as described above. Particular examples may include $Ni^{2+}$, $Zn^{2+}$, $Fe^{3+}$ or $Ga^{3+}$, among others.

Linker 30 generally comprises any molecule that links luminescent or enzymatically functional molecule 26 to metal ion 28. The linker may link metal ion 28 to the luminescent or enzymatically functional molecule 26 through any kind of molecular interaction, including ionic linkages, covalent chemical bonds, or a combination of ionic and covalent interactions. An example of such a linker linking ruthenium (Ru) to $Fe^{3+}$ through an ionic interaction is $Ru-C-N-(CH_2COO^-)_2\ Fe^{3+}$.

The presence of luminescent or enzymatically functional molecule 26 may be detected by any appropriate means. If molecule 26 is luminescent, it may be detected by any means capable of detecting luminescence, including a microscope, a microplate reader, or a spectrophotometer or spectrofluorimeter designed to detect luminescence, luminescence resonance energy transfer, and/or luminescence polarization, among others. If molecule 26 is enzymatically functional, it may generally be detected by its catalysis of any reaction that produces a detectable change. This change may be detected by any appropriate means. An example of such a reaction is the oxidation of a chromogen in the presence of a peroxide catalyzed by a peroxidase, which causes a visible color change. Examples of enzymes that may catalyze such reactions include all or part of horseradish peroxidase or variants thereof. Examples of chromogens that may participate in such reactions include 3,3',5,5'-tetramethylbenzidine and 2,2'-azino-bis(3-ethylbenzthiazoline-6-sulfonic acid). The color change may be detected by, for example, a microscope, a microplate reader, a spectrophotometer, or the human eye.

### III. *Methods for Purifying and Labeling Any Kind of Molecule*

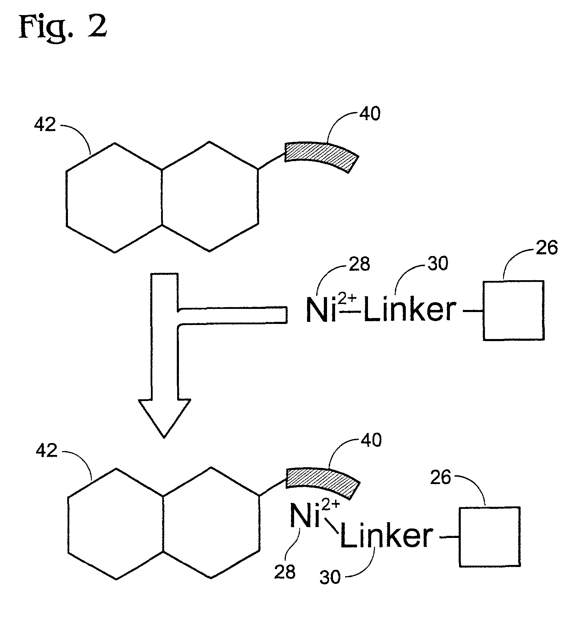

Figure 2 shows an alternate embodiment of the invention in which any kind of molecule can be labeled. Protein tag 40 is covalently conjugated to a molecule of interest 42, which can be any kind of molecule. The covalent conjugate of protein tag 40 and molecule of interest 42 can be purified as described above for hybrid protein 20, 22. Luminescent or enzymatically functional molecule 26 conjugated to metal ion 28 is added to the conjugate of protein tag 40 and molecule of interest 42. The binding of protein tag 40 with metal ion 28 links luminescent or enzymatically functional molecule 26 to molecule of interest 42. Luminescent or enzymatically functional molecule 26 may be detected as described above.

Alternatively, protein tag 40 is luminescent. It may be covalently conjugated to molecule of interest 42, thus labeling it. Examples of such luminescent protein tags may include all or part of Green Fluorescent Protein (GFP) or luminescent variants of GFP.

### IV. *Overview of Selected Methods for Detecting Conformational State and/or Proximity of Molecules*

The invention provides assay methods for detecting the conformational state and/or proximity of species or molecules, including both the association (e.g., binding) and dissociation (e.g., cleavage) of molecules.

In one aspect of the invention, a suitably labeled substance is exposed to a condition that is capable of causing the molecule to change conformational state. Here, conformation state refers to the spatial arrangement of atoms, molecules, or subunits within a molecule or supramolecular assembly. These conditions may include elevated temperature, which can result in loss of quaternary and tertiary structure in proteins, and a loss of hybridization in DNA or RNA duplexes. Alternatively, these conditions may include the presence of a complementary member of a labeled specific binding pair member, which may result in a change of conformation to accommodate binding of the specific binding pair members, such as the presence of the complement to a labeled strand of DNA or RNA.

In another aspect of the invention, a suitably labeled substance or set of substances is exposed to a condition that is capable of causing the substance or set of substances to dissociate or associate, as appropriate. These assays may change the separation between energy transfer partners, increasing or decreasing the amount of luminescence energy transfer. Alternatively, or in addition, these assays may change the relative size of a species, increasing or decreasing its luminescence polarization. In a typical assay, the labeled substance is exposed to the condition for a period of time sufficient for association or dissociation to occur, and then the appropriate change in luminescence signal is detected. The assay may be termed a continuous assay if the detecting step is conducted during the association/dissociation step.

Association assays typically measure the binding of assay components, such as specific binding partners and/or a probe and an unknown whose presence, concentration, and/or activity is being measured.

Dissociation assays typically measure the degradation or hydrolysis of a labeled substance. The condition may involve exposure to a chemical agent capable of cleaving the species, or to a condition of elevated temperature at which the species is unstable with respect to degradation. The condition may be the presence of an enzyme such as a nuclease (e.g., exonuclease or endonuclease) or protease or carbohydrase capable of cleaving the labeled substance. In enzymatic dissociation, the labeled substance typically is combined with the appropriate enzyme in a way that facilitates contact of the enzyme and the labeled substance under conditions suitable for hydrolysis to occur, for example, by mixing a solution of the labeled substance with a sample that contains or is suspected of containing an enzyme or multiple enzymes. The enzyme optionally is free in solution, enclosed within a biological structure, extracted from a biological structure, and/or immobilized in or on a solid or semi-solid matrix, such as a membrane or the surface of a microwell.

The condition(s) to which the substance or substances are exposed during an assay generally include any mode or state of being capable of bringing about a change in the parameter under observation. These conditions may involve changes in temperature, pH, ionic strength, osmolarity, solute composition, and/or solvent composition, among others, and/or exposure to enzymes, detergents, and/or physical agitation, among others (e.g., shaking, stirring, sonication, etc.).

The labeled substance generally is exposed to the condition under conditions that allow the expected or desired change to occur. Thus, for enzymatic activity, the substance is combined with the enzyme under suitable conditions that allow for hydrolysis. These conditions may be affected by temperature, pH and changes in composition of the medium. Such conditions may be adjusted to accelerate or decelerate the enzymatic reaction or to favor or hinder a particular enzymatic reaction.

The labeled substance generally is exposed to the condition for a period of time necessary to allow the expected or desired change to occur. Thus, results may be monitored continuously, intermittently, or at an endpoint of the expected reaction time. Continuous and intermittent monitoring is useful in assays, such as hydrolytic assays, that result in a continuous change in luminescence properties.

*V. Energy Transfer Methods for Detecting Conformational State and/or Proximity of Molecules*

Energy transfer is the transfer of luminescence energy from a donor luminophore to an acceptor without emission by the donor. In energy transfer assays, a donor luminophore is excited from a ground state into an excited state by absorption of a photon. If the donor luminophore is sufficiently close to an acceptor, excited-state energy may be transferred from the donor to the acceptor, causing donor luminescence to decrease and acceptor luminescence to increase (if the acceptor is luminescent). The efficiency of this transfer is very sensitive to the separation R between donor and acceptor, decaying as $1/R^{-6}$. Energy transfer assays use energy transfer to monitor the proximity of donor and acceptor.

Donor/acceptor pairs of luminescent molecules generally comprise any pair of molecules in which one molecule (the donor) is capable of transferring excited-state energy to the other molecule (the acceptor). The rate of energy transfer may depend upon the extent of spectral overlap of the emission spectrum of the donor with the absorption spectrum of the acceptor, the quantum yield of the donor, the relative orientation of the donor and acceptor transition dipoles, and the distance between the donor and acceptor molecules. Typically, the donor is excited using light that is not significantly absorbed by the acceptor. Examples of donor/acceptor pairs may include the following: carboxyfluorescein succinimidyl ester and Texas Red sulfonyl chloride, or fluorescein and rhodamine, among others.

Some energy transfer assays focus on an increase in energy transfer as donor and acceptor are brought into proximity. These assays may be used to monitor binding, as between two molecules X and Y to form a complex X: Y. Here, colon (:) represents a noncovalent interaction. In these assays, one molecule is labeled with a donor D, and the other molecule is labeled with an acceptor A, such that the interaction between X and Y is not altered appreciably. Independently, D and A may be covalently attached to X and Y, or covalently attached to binding partners of X and Y.

Other energy transfer assays focus on a decrease in energy transfer as donor and acceptor are separated. These assays may be used to monitor cleavage, as by hydrolytic digestion of doubly labeled substrates (peptides, nucleic acids). In a typical application, two ends of a polypeptide might be labeled with D and A, so that cleavage of the polypeptide by an endopeptidase will separate D and A and thereby reduce energy transfer. Energy transfer assays are also useful for monitoring changes in conformation by a doubly labeled substrate, such as changes in quaternary structure of a protein, or formation of a g-quartet by a nucleic acid polymer, where the change in conformation changes the distance between D and A.

Energy transfer between D and A may be monitored in various ways. For example, energy transfer may be monitored by observing an energy-transfer induced decrease in the emission intensity of D and increase in the emission intensity of A (if A is a luminophore). Energy transfer also may be monitored by observing an energy-transfer induced decrease in the lifetime of D and increase in the apparent lifetime of A.

In some assays, a long-lifetime luminophore may be used as a donor, and a short-lifetime luminophore may be used as an acceptor. Suitable long-lifetime luminophores include metal-ligand complexes containing ruthenium, osmium, etc., and lanthanide chelates containing europium, terbium, etc. In time-domain assays, the donor is excited using a flash of light having a wavelength near the excitation maximum of D. Next, there is a brief wait, so that electronic transients and/or short-lifetime background luminescence can decay. Finally, donor and/or acceptor luminescence intensity is detected and integrated. In frequency-domain assays, the donor is excited using time-modulated light, and the phase and/or modulation of the donor and/or acceptor emission is monitored relative to the phase and/or modulation of the excitation light. In both assays, donor luminescence is reduced if there is energy transfer, and acceptor luminescence is observed only if there is energy transfer.

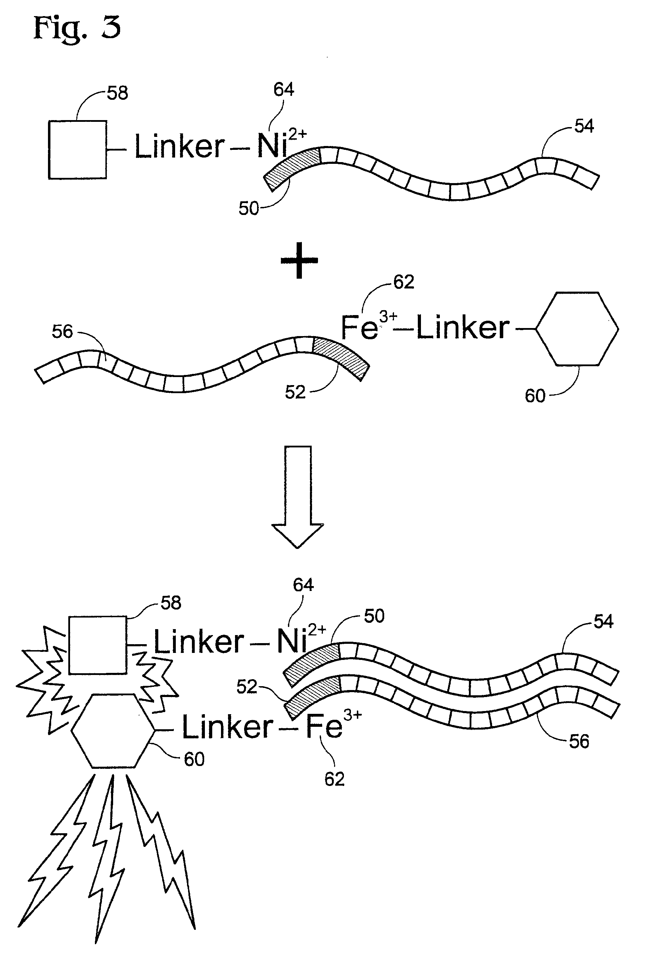

For example, Figure 3 shows a method for determining whether two labeled molecules are in physical proximity to each other (including bound to each other). Two molecules, each comprising a protein tag 50, 52 and a molecule of interest 54, 56, are luminescently labeled by the methods shown in Figures 1 and/or 2 using luminescent molecules 58, 60 linked to metal ions 62, 64 or luminescent protein tags. The two molecules of interest 54, 56 may be the same or different, but luminescent molecules 58, 60 (or protein tags 50, 52, if they are luminescent) used to label them should comprise an appropriately matched donor/acceptor (60/58 or 52/50) pair, between which luminescence resonance energy transfer can occur. The two labeled molecules of interest 54, 56 may be incubated together under conditions that allow binding or interaction. Donor luminescent molecule 60 (or protein tag 52) may be excited by light of an appropriate wavelength. The resulting heightened energy state of donor molecule 60 (or protein tag 52) may be transferred to acceptor molecule 58 (or protein tag 50) if it is physically close enough. For common donor/acceptor pairs, donor 60 and acceptor 58 (or protein tags 52 and 50) should be within about 50-100 nm for energy transfer to occur. If energy transfer does occur, it may be detected by a decrease in the luminescence intensity and/or luminescence lifetime of donor 60 (or protein tag 52) and/or by an increase in the luminescence intensity of acceptor 58 (or protein tag 50), if the acceptor is luminescent. The detection of energy transfer between donor 60 and acceptor 58 (or protein tags 52 and 50) may indicate an interaction between molecules of interest 54, 56, including binding.

Protein tags 50 and 52 generally comprise luminescent peptides or peptides that can bind to metal ions. If protein tags 50 and 52 comprise luminescent peptides, then these peptides may be capable of acting as a donor/acceptor pairs for the purposes of detection of binding by luminescence resonance energy transfer. Examples of such peptides may include two variants of Green Fluorescent Protein (GFP), such as EBFP and EGFP. Other examples also are possible. If protein tags 50 and 52 can bind to metal ions, examples may include polyhistidine, peptides in which every other residue is a histidine, or phosphorylated peptides of any sequence.

Molecules of interest 54, 56 generally comprise any molecules to which protein tag 50, 52 may be chemically conjugated, such as proteins, nucleic acids, carbohydrates, sugars, or nucleotides, as discussed above.

Luminescence energy transfer may be measured using any appropriate apparatus and methods, including apparatus and methods described in patents, patent applications, and nonpatent references cited above under Cross-References and incorporated herein by reference.

### VI. Polarization Methods for Detecting Conformational State and/or Proximity of Molecules

Luminescence polarization assays involve the absorption and emission of polarized light, and typically are used to study molecular rotation. (Polarization describes the direction of light's electric field, which generally is perpendicular to the direction of light's propagation.)

In a luminescence polarization assay, specific molecules within a composition are labeled with one or more luminophores. The composition then is illuminated with polarized excitation light, which preferentially excites luminophores having absorption dipoles aligned parallel to the polarization of the excitation light. These molecules subsequently decay by preferentially emitting light polarized parallel to their emission dipoles. The extent to which the total emitted light is polarized depends on the extent of molecular reorientation during the time interval between luminescence excitation and emission, which is termed the luminescence lifetime, $\tau$. The extent of molecular reorientation in turn depends on the luminescence lifetime and the size, shape, and environment of the reorienting molecule. Thus, luminescence polarization assays may be used to quantify binding reactions and enzymatic activity, among other applications. In particular, molecules commonly rotate via diffusion with a rotational correlation time $\tau_{rot}$ that is proportional to their size. Thus, during their luminescence lifetime, relatively large molecules will not reorient significantly, so that their total luminescence will be relatively polarized. In contrast, during the same time interval, relatively small molecules will reorient significantly, so that their total luminescence will be relatively unpolarized.

The relationship between polarization and intensity is expressed by the following equation:

$$P = \frac{I_{\parallel} - I_{\perp}}{I_{\parallel} + I_{\perp}}$$

Here, P is the polarization, $I_{\parallel}$ is the intensity of luminescence polarized parallel to the polarization of the excitation light, and $I_{\perp}$ is the intensity of luminescence polarized perpendicular to the polarization of the excitation light. P generally varies from zero to one-half for randomly oriented molecules (and zero to one for aligned molecules). If there is little rotation between excitation and emission, $I_{\parallel}$ will be relatively large, $I_{\perp}$ will be relatively small, and P will be close to one-half. (P may be less than one-half even if there is no rotation; for example, P will be less than one if the absorption and emission dipoles are not parallel.) In contrast, if there is significant rotation between absorption and emission, $I_{\parallel}$ will be comparable to $I_{\perp}$, and P will be close to zero. Polarization often is reported in milli-P (mP) units (1000×P), which for randomly oriented molecules will range between 0 and 500, because P will range between zero and one-half.

Polarization also may be described using other equivalent quantities, such as anisotropy. The relationship between anisotropy and intensity is expressed by the following equation:

$$r = \frac{I_{\parallel} - I_{\perp}}{I_{\parallel} + 2I_{\perp}}$$

Here, r is the anisotropy. Polarization and anisotropy include the same information, although anisotropy may be more simply expressed for systems containing more than one luminophore. In the description and claims that follow, these terms may be used interchangeably, and a generic reference to one should be understood to imply a generic reference to the other.

The relationship between polarization, luminescence lifetime, and rotational correlation time is expressed by the Perrin equation:

$$\left(\frac{1}{P}-\frac{1}{3}\right)=\left(\frac{1}{P_0}-\frac{1}{3}\right)\cdot\left(1+\frac{\tau}{\tau_{rot}}\right)$$

Here, $P_0$ is the polarization in the absence of molecular motion (intrinsic polarization), $\tau$ is the luminescence lifetime (inverse decay rate), as described above, and $\tau_{rot}$ is the rotational correlation time (inverse rotational rate), as described above.

The Perrin equation shows that luminescence polarization assays are most sensitive when the luminescence lifetime and the rotational correlation time are similar. Rotational correlation time is proportional to molecular weight, increasing by about 1 nanosecond for each 2,400 Dalton increase in molecular weight (for a spherical molecule). For shorter lifetime luminophores, such as fluorescein, which has a luminescence lifetime of roughly 4 nanoseconds, luminescence polarization assays are most sensitive for molecular weights less than about 40,000 Daltons. For longer lifetime probes, such as Ru(bpy)$_2$dcbpy (ruthenium 2,2'-dibipyridyl 4,4'-dicarboxyl-2,2'-bipyridine), which has a lifetime of roughly 400 nanoseconds, luminescence polarization assays are most sensitive for molecular weights between about 70,000 Daltons and 4,000,000 Daltons.

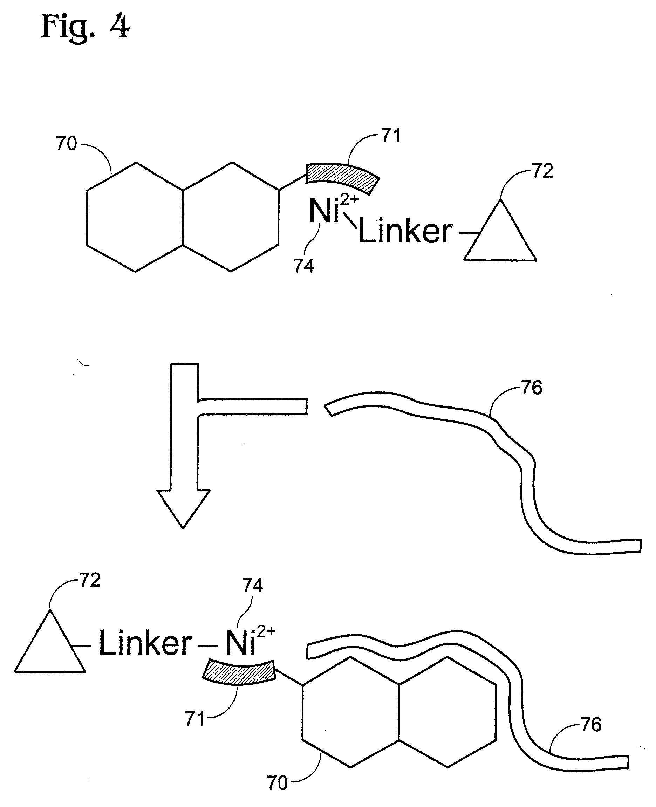

Figure 4 shows a method for determining whether a small molecule binds to a macromolecule, such as a protein, using luminescence polarization. A small molecule 70 conjugated to a protein tag 71 is labeled as described in Figures 1 and/or 2 with a luminescent molecule 72 conjugated to a metal ion 74 or a luminescent protein tag 71. Luminescence polarization is measured. A macromolecule 76 is added, and the mixture is incubated to allow binding. Luminescence polarization is measured again to determine whether the addition of macromolecule 76 has caused a change in luminescence polarization. An increase in luminescence polarization indicates that macromolecule 76 is restricting the rotation of small molecule 70, possibly by binding to it.

Small molecule 70 generally comprises any molecule that is sufficiently smaller than macromolecule 76 to experience an observable change in luminescence polarization when macromolecule 76 binds to small molecule 70. Examples of such small molecules may include carbohydrates, sugars, metals, peptides, or oligonucleotides, among others.

Conversely, macromolecule 76 generally comprises any molecule that is sufficiently larger than small molecule 70 to induce an observable change in the polarization of the small molecule when it binds with the macromolecule. Examples of such macromolecules may include proteins, RNA, or DNA, among others. Macromolecule 76 also may comprise a relatively small molecule used in conjunction with "mass labeling," as described in WO 00/23785, which is incorporated herein by reference.

Protein tag 71 generally comprises a luminescent peptide or a peptide P that can bind to metal ions. Examples may include the following polyhistidine, peptides in which every other residue is a histidine, phosphorylated peptides of any sequence, or all or part of GFP or variants thereof.

Luminescent molecule 72 generally comprises any luminescent molecule with an appropriate intrinsic polarization and luminescence lifetime for showing a change in luminescence polarization when labeled small molecule 70, 71, 72, 74 is bound by macromolecule 76.

Metal ion 74 generally comprises any metal ion in any oxidation state that can bind to protein tag 71.

Luminescence polarization may be measured using any appropriate apparatus and methods, including apparatus and methods described in patents, patent applications, and nonpatent references cited above under Cross-References and incorporated herein by reference.

The disclosure set forth above may encompass multiple distinct inventions with independent utility. While each of these inventions has been disclosed in its preferred form, the specific embodiments thereof as disclosed and illustrated herein are not to be considered in a limiting sense as numerous variations are possible. The subject matter of the inventions includes all novel and nonobvious combinations and subcombinations of the various elements, features, functions and/or properties disclosed herein. Similarly, where the claims recite "a" or "a first" element or the equivalent thereof, such claims should be understood to include incorporation of one or more such elements, neither requiring nor excluding two or more such elements. It is believed that the following claims particularly point out certain combinations and subcombinations that are directed to one of the disclosed inventions and are novel and nonobvious. Inventions embodied in other combinations and subcombinations of features, functions, elements and/or properties may be claimed through amendment of the present claims or presentation of new claims in this or a related application. Such amended or new claims, whether they are directed to a different invention or directed to the same invention, whether different, broader, narrower or equal in scope to the original claims, are also regarded as included within the subject matter of the inventions of the present disclosure.

**Claims**

1. A product having the formula

T-P-M-L

where T is a species or molecule;
M is a metal ion;
P is a peptide or protein that binds the metal ion; and
L is a luminescent label.

2. The product of claim 1, where the species or molecule is an amino acid, a peptide, a protein, a polysaccharide, a nucleotide, an oligonucleotide, a nucleic acid polymer.

3. The product of claim 1, where the species or molecule is a drug, a lipid, a lipid assembly, a non-biological organic polymer, or a polymeric microparticle.

4. The product of claim 1, where the species or molecule is a peptide, protein, polysaccharide, nucleotide, oligonucleotide, or nucleic acid polymer.

5. The product of any of claims 1 to 4, where L comprises a luminophore that is covalently bound to the metal ion.

6. The product of claim 5, where L comprises a $Eu^{3+}$ or a $Tb^{3+}$ ion.

7. The product of claim 5, where the luminophore is a fluorophore.

8. The product of claim 7, where the fluorophore is a xanthene dye, for example a fluorescein, a rosamine, a rhodamine, or a rhodol.

9. The product of claim 7, where the fluorophore is a benzimidazole, a phenoxazine, an ethidium, a propidium, an acridine, a styryl dye, a carbocyanine, a merocyanines, a coumarin, a pyrene, a chrysene, a stilbene, a carbazine, an anthracene, a naphthalene, a dipyrromethentboron difluoride, or a dibenzodipyrromethenboron difluoride.

10. The product of any of claims 1 to 9, where P includes a peptide sequence that incorporates multiple histidine residues and M is a $Ni^{2+}$ ion.

11. The product of claim 10 wherein the fluorescent dye is linked to the metal ion through a nitrilotriacetic acid moiety.

12. The product of any of claims 1 to 9, where P includes a phosphorylated peptide sequence and M is a $Ga^{3+}$ or a $Fe^{3+}$ ion.

13. The product of any of claims 1 to 12, where T includes a member of a specific binding pair, the product further comprising a complementary member of the specific binding pair that is associated with T.

14. The product of claim 13, where the complementary specific binding pair member is labeled with a dye that is able to accept luminescence energy transfer from L, or that is able to donate luminescence energy transfer to L.

15. The product of claim 13, where the complex of the complementary specific binding pair member with the product exhibits detectably different luminescence polarization than that exhibited by the product alone.

16. The product of any of claims 1 to 15, where P is chemically conjugated to T.

17. A composition of matter, comprising:

a luminescence energy donor; and
a luminescence energy acceptor;

where the energy acceptor is capable of accepting energy transfer from the energy donor, and where at least one of the energy donor and energy acceptor has the formula

P-M-L

where

M is a metal ion;
P is a peptide or protein that binds the metal ion; and
L is a luminescent label.

18. The composition of matter of claim 17, where the energy donor and energy acceptor are covalently bound to a species or molecule.

19. The composition of matter of claim 18, where the molecule is susceptible to cleavage by exposure to an appropriate environmental condition, where such cleavage would decrease the degree of energy transfer from the energy donor to the energy acceptor.

20. The composition of matter of claim 19, where the appropriate environmental condition comprises the presence of a selected substance.

21. The composition of matter of claim 20, where the selected substance is an appropriate enzyme.

22. The composition of matter of any of claims 17 to 21, where the energy donor is associated with a first member of a specific binding pair, and the energy acceptor is associated with a second member of the specific binding pair, where the first and second specific binding pair members are complementary and are associated with each other such that there is detectable energy transfer from the energy donor to the energy acceptor.

23. The composition of matter of any of claims 17 to 22 which further includes the feature(s) recited in one or more of claims 7 to 12.

24. A method of labeling a species or molecule, comprising the steps of:

a) conjugating a polypeptide to the species or molecule, where the polypeptide is capable of binding a selected metal ion;
b) preparing a label comprising a luminophore that is covalently linked to the selected metal ion; and
c) combining the prepared label with the polypeptide conjugate, under conditions where the polypeptide will bind the selected metal ion.

25. The method of claim 24, where the polypeptide is conjugated to the species or molecule via a terminal carboxylic acid group or a terminal amine group.

26. The method of claim 24 or claim 25, where the species or molecule is a member of a specific binding pair.

27. The method of claim26, where the molecule is a peptide, a protein, an oligonucleotide, or a nucleic acid polymer.

28. The method of any of claims 24 to 27, which further includes the features recited in claim 10 or in claims 10 and 11.

29. A method of detecting energy transfer in a sample, comprising the steps of:

a) contacting the sample with a luminescence energy donor and a luminescence energy acceptor, where the energy acceptor is capable of accepting energy transfer from the energy donor;
b) illuminating the sample at a wavelength suitable for excitation of the energy donor; and
c) measuring an amount of luminescence energy transfer from the energy donor to the energy acceptor;

where at least one of the energy donor and energy acceptor has the formula

P-M-L

where

M is a metal ion;
P is a peptide or protein that binds the metal ion; and
L is a luminescent label.

30. The method of claim 29, wherein the step of measuring the amount of luminescence energy transfer from the energy donor to the energy acceptor comprises measuring a decrease in energy donor luminescence or measuring an increase in energy acceptor luminescence.

31. The method of claim 29, wherein the step of measuring the amount of luminescence energy transfer from the energy donor to the energy acceptor comprises measuring a decrease in energy donor luminescence lifetime.

32. The method of any of claims 29 to 31, where the energy donor and the energy acceptor are bound to a first and second member of a specific binding pair, respectively, and further comprising the step of correlating the amount of luminescence energy transfer with the association of the first and second members of the specific binding pair.

33. The method of any of claims 29 to 31, where the energy donor and the energy acceptor are bound to first and second portions of the same species or molecule such that luminescence energy transfer occurs between the energy donor and the energy acceptor, further comprising correlating the amount of luminescence energy transfer with the dissociation of the two portions of the species or molecule.

34. A method of detecting dissociation of a molecule, comprising the steps of:

a) providing a species or molecule that is labeled with both a luminescence energy donor and a luminescence energy acceptor such that luminescence energy transfer occurs between the energy donor and the energy acceptor;
b) exposing the species or molecule to a condition that is capable of dissociating the substance such that the energy donor and the energy acceptor are no longer capable of luminescence energy transfer;
c) illuminating the sample at a wavelength suitable for excitation of the energy donor; and
d) measuring an amount of luminescence energy transfer from the energy donor to the energy acceptor; and
e) correlating the amount of luminescence energy transfer from the energy donor to the energy acceptor with the dissociation of the species or molecule;

where at least one of the energy donor and energy acceptor has the formula

P-M-L

where

M is a metal ion;
P is a peptide or protein that binds the metal ion; and
L is a luminescent label.

35. The method of claim 34, where the molecule is a peptide, a protein, an oligonucleotide, or a nucleic acid polymer.

36. The method of claim 34, where the molecule is an enzyme substrate, and the step of exposing the molecule to a condition that is capable of dissociating the molecule comprises exposing the substance to an appropriate enzyme.

37. A method of detecting a change in conformation of a species or molecule, comprising the steps of:

a) providing a species or molecule that is labeled with both a luminescence energy donor and a luminescence energy acceptor, where the energy donor is capable of energy transfer to the energy acceptor;

b) illuminating the sample at a wavelength suitable for excitation of the energy donor;

c) measuring a first amount of luminescence energy transfer from the energy donor to the energy acceptor;

d) exposing the species or molecule to a condition that is capable of causing the molecule to change conformation;

e) illuminating the sample at a wavelength suitable for excitation of the energy donor;

f) measuring a change in the luminescence energy transfer from the energy donor to the energy acceptor; and

g) correlating the change in luminescence energy transfer from the energy to the energy acceptor with the change in conformation of the molecule;

where at least one of the energy donor and energy acceptor has the formula

P-M-L

where

M is a metal ion;

P is a peptide or protein that binds the metal ion; and

L is a luminescent label.

38. The method of claim37, where the molecule is a peptide, a protein, an oligonucleotide, or a nucleic acid polymer.

39. A method of detecting an analyte, comprising the steps of:

a) measuring the luminescence polarization of a productas defined by any of claims 1 to 16;

b) contacting the compound with a sample thought to contain the analyte, where the analyte is the complementary member of the specific binding pair;

c) incubating the sample for a time sufficient for the compound to form a complex with the analyte;

d) measuring the luminescence polarization of the complex; and

e) correlating the luminescence polarization of the complex with the presence of the analyte.

40. A kit, comprising a product as defined by any of claims 1 to 16.

41. The kit of claim 40, further comprising at least one additional reagent.

42. The kit of claim 41, wherein the additional reagent is a buffering agent, a luminescence calibration standard, an enzyme, an enzyme substrate, a nucleic acid stain, or a labeled antibody.

43. The kit of any of claims 40 to 42, wherein the kit is configured for use in conjunction with a sample in a multiwell microplate or a microfluidic chip.

# Fig. 1

# Fig. 2

# Fig. 3

# Fig. 4

**European Patent Office**

**EUROPEAN SEARCH REPORT**

Application Number

EP 01 31 0076

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 952 236 A (GE ZHENGFANG ET AL) 14 September 1999 (1999-09-14)<br><br>* abstract *<br>* column 6, line 39-43 *<br>* column 6, line 65-67 *<br>* figure 2 * | 1,3, 16-18, 29,34-38 | G01N33/58 |
| A | US 5 808 003 A (SUBRAMANIAN RAMASWAMY ET AL) 15 September 1998 (1998-09-15)<br>* the whole document * | 1-43 | |

**TECHNICAL FIELDS SEARCHED (Int.Cl.7)**

G01N

The present search report has been drawn up for all claims

| Place of search | Date of completion of the search | Examiner |
|---|---|---|
| MUNICH | 22 April 2002 | Weijland, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 01 31 0076

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2002

| Patent document cited in search report | | Publication date | Patent family member(s) | | Publication date |
|---|---|---|---|---|---|
| US 5952236 | A | 14-09-1999 | US | 6225127 B1 | 01-05-2001 |
| | | | US | 6197258 B1 | 06-03-2001 |
| US 5808003 | A | 15-09-1998 | US | 5292868 A | 08-03-1994 |
| | | | US | 5204448 A | 20-04-1993 |
| | | | US | 5488126 A | 30-01-1996 |
| | | | US | 5583219 A | 10-12-1996 |
| | | | AT | 128035 T | 15-10-1995 |
| | | | AU | 638757 B2 | 08-07-1993 |
| | | | AU | 6427590 A | 07-01-1991 |
| | | | CA | 2033086 A1 | 27-11-1990 |
| | | | DE | 69022542 D1 | 26-10-1995 |
| | | | DE | 69022542 T2 | 02-05-1996 |
| | | | DK | 429644 T3 | 22-01-1996 |
| | | | EP | 0429644 A1 | 05-06-1991 |
| | | | EP | 0661267 A1 | 05-07-1995 |
| | | | ES | 2080160 T3 | 01-02-1996 |
| | | | FI | 104409 B1 | 31-01-2000 |
| | | | IE | 68411 B1 | 12-06-1996 |
| | | | IE | 68411 L | 26-11-1990 |
| | | | JP | 2865112 B2 | 08-03-1999 |
| | | | JP | 4500389 T | 23-01-1992 |
| | | | KR | 199892 B1 | 15-06-1999 |
| | | | KR | 186799 B1 | 15-05-1999 |
| | | | WO | 9014881 A2 | 13-12-1990 |
| | | | ZA | 9004047 A | 25-09-1991 |
| | | | AU | 692224 B2 | 04-06-1998 |
| | | | AU | 1597595 A | 01-08-1995 |
| | | | EP | 0738283 A1 | 23-10-1996 |
| | | | FI | 962772 A | 05-07-1996 |
| | | | JP | 9507488 T | 29-07-1997 |